# EUROPÄISCHE PATENTSCHRIFT

(11) **EP 0 364 794 B1**
(45) Veröffentlichungstag und Bekanntmachung des Hinweises auf die Patenterteilung: **08.06.1994**
(21) Anmeldenummer: 89118125.7
(22) Anmeldetag: 29.09.1989
(51) Int. Cl.: G02B 21/06

(54) **Beleuchtungseinrichtung für Operationsmikroskope**
Illumination device for an operation microscope
Dispositif d'illumination pour un microscope d'opération

(30) Priorität: 05.10.1988 DE 3833877
(43) Veröffentlichungstag der Anmeldung: 25.04.1990
(73) Patentinhaber: Firma Carl Zeiss, D-73446 Oberkochen (DE)
(72) Erfinder: Sander, Ulrich, Dr., D-7082 Oberkochen (DE); Biber, Klaus, D-7080 Aalen (DE)

(56) Entgegenhaltungen:
- DE-A- 3 200 938
- DE-A- 3 516 271
- DE-U- 8 713 356
- DE-U- 8 802 175
- DE-U- 8 802 996
- US-A- 4 511 223

## Beschreibung

Die Erfindung betrifft eine Beleuchtungseinrichtung für Operationsmikroskope mit einem Hauptobjektiv für die beiden stereoskopischen Beobachtungsstrahlengänge und mit einer Lichtquelle, welcher ein Lichtleiter nachgeordnet ist.

Bei Operationen im Bereich der Hals-Nasen-Ohren-Medizin, der Neurologie und der Gynäkologie werden Operationsmikroskope benötigt, die eine Einstellung sowohl großer als auch sehr kleiner Leuchtfelder erlauben.

Aus dem DE-GM 87 13 356.3 ist eine Beleuchtungseinrichtung für Operationsmikroskope bekannt, die es erlaubt, mit einem unveränderlichen Optiksystem unterschiedliche Leuchtfelddurchmesser zu realisieren. Nachteilig bei dieser bekannten Beleuchtungseinrichtung ist, daß ihr Dehnungsfaktor den operationsspezifischen Anforderungen nicht voll genügt.

Der Erfindung liegt die Aufgabe zugrunde, eine Beleuchtungseinrichtung für Operationsmikroskope mit einem genügend großen, den operationsspezifischen Anforderungen voll entsprechendem Dehnungsfaktor des Leuchtfeldes anzugeben.

Diese Aufgabe wird erfindungsgemäß durch die Merkmale des Anspruchs 1 gelöst. Hierbei ist zwischen dem Hauptobjektiv des Operationsmikroskopes und der Lichtaustrittsfläche des Lichtleiters der Beleuchtungseinrichtung ein entlang der optischen Achse verschiebbares System vorgesehen, das aus einer Sammellinse und einer asphärischen Linse sowie einer Blende besteht.

In einem vorteilhaften Ausführungsbeispiel der Erfindung besteht zwischen dem Durchmesser der Blende, dem Verschiebeweg des optischen Systems und dem eingestellten Leuchtfelddurchmesser eine lineare Korrelation, die beispielsweise durch einen getriebegesteuerten Verschiebemechanismus bewirkt wird.

Durch einen kurvengesteuerten Verschiebemechanismus kann in einem anderen Ausführungsbeispiel zwischen den oben genannten Beleuchtungsparametern eine nichtlineare Korrelation hergestellt werden. Dabei wird die Einstellung der Beleuchtung vorteilhaft so vorgenommen, daß zunächst die Blende teilweise geöffnet wird und daß bei der weiteren Öffnung der Blende diese zusammen mit dem optischen System axial verschoben wird.

Zweckmäßig ist die Beleuchtungseinrichtung so ausgebildet, daß zur Justierung der Lichtleiter relativ zur Blende verschoben werden kann. Damit läßt sich die Ausleuchtung des Objektfeldes wahlweise homogen oder mittenbetont einstellen, wobei diese Einstellung bei der Wahl der Größe des ausgeleuchteten Feldes erhalten bleibt.

Die mit der Erfindung erzielten Vorteile bestehen insbesondere darin, daß das Beleuchtungssystem extrem kurzbauend ist, auch bei großen Leuchtfeldern eine gewünschte Ausleuchtung liefert und bei kleinen Leuchtfeldern eine gute Randschärfe sowie eine hohe Beleuchtungsstärke garantiert, was insbesondere bei Operationen in engen Kanälen bedeutsam ist, weil in diesen Fällen das von zu großen Leuchtfeldern verursachte Streulicht den Kontrast im Arbeitsfeld verschlechtert.

Ein Ausführungsbeispiel der Erfindung ist in den Figuren 1 - 3 der beigefügten Zeichnung dargestellt und wird im folgenden näher beschrieben.

Es zeigen
- Figur 1: einen Schnitt durch die nach der Erfindung ausgebildete Beleuchtungseinrichtung;
- Figur 2: einen Schnitt entlang der Linie II-II der Fig. 1;
- Figur 3: die Anordnung der Beleuchtungseinrichtung nach Fig. 1 an einem Operationsmikroskop.

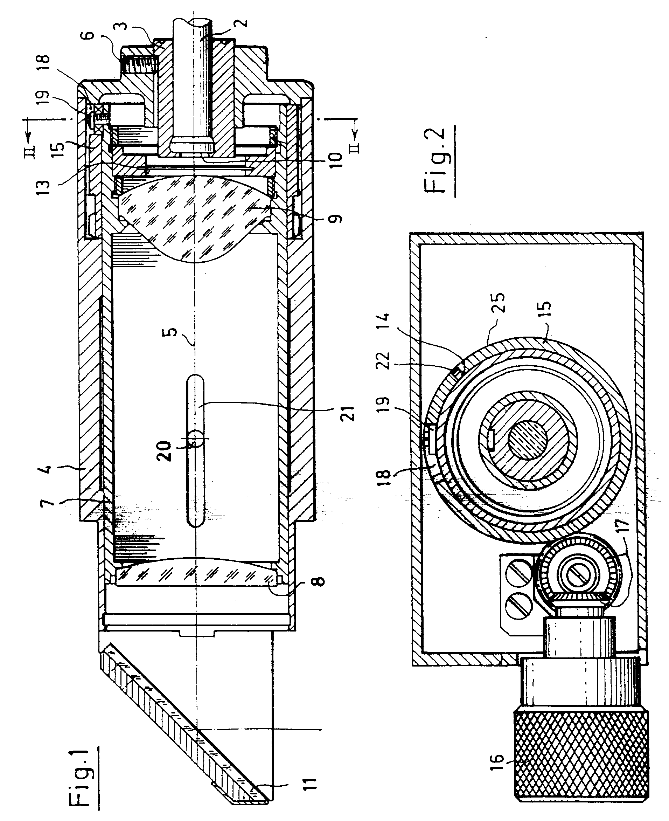

In Fig. 1 ist mit (2) ein Faserlichtleiter bezeichnet, in den das Licht einer nicht eingezeichneten Lichtquelle eingespeist ist. Der Lichtleiter (2) ist in einem zylindrischen Gehäuse (3) gefaßt, das im Gehäuse (4) angeordnet ist. Zu Justierzwecken ist der Lichtleiter (2) mit seinem Gehäuse (3) in Richtung der optischen Achse (5) verschiebbar, wobei die Schraube (6) zur Fixierung der Justiereinstellung dient.

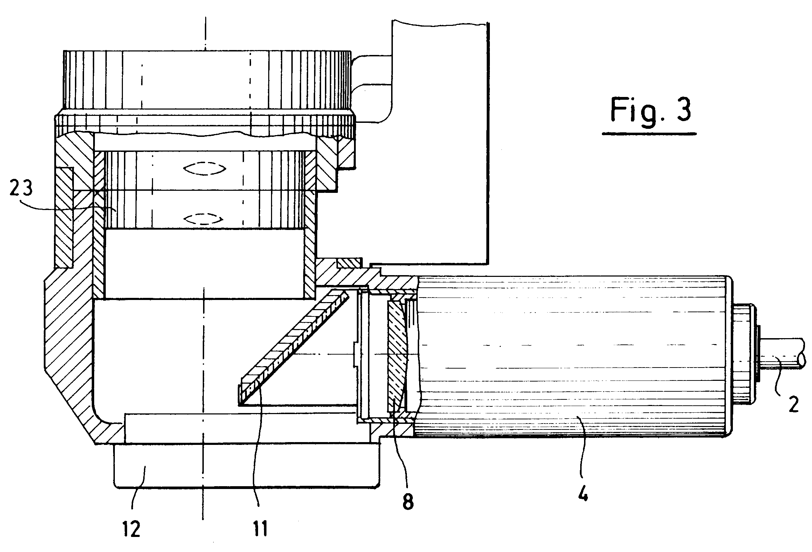

Im Gehäuse (4) ist eine Fassung (7) in Richtung der optischen Achse (5) verschiebbar gelagert. Die Fassung (7) enthält ein optisches System, bestehend aus einer Sammellinse (8) und einer asphärischen Linse (9). Dieses System bildet die Lichtaustrittsfläche (10) des Lichtleiters (2) über einen Umlenkspiegel (11) und das aus Fig. 3 ersichtliche Hauptobjektiv (12) des Operationsmikroskops auf das Operationsfeld ab.

Die Fassung (7) enthält das optische System (8, 9) sowie eine Blende (13). Diese Blende kann als Kreisblende oder als Spaltblende oder als kombinierte Kreis-Spaltblende ausgeführt sein. Über den in Fig. 2 dargestellten Hebel (14) wird die Öffnung der Blende (13) verstellt.

Zwischen dem Gehäuse (4) und der Fassung (7) ist ein zylindrischer Ring (15) drehbar angeordnet. Der in Fig. 2 dargestellte Drehknopf (16) dient zur Drehung des Ringes (15). Diese Betätigung erfolgt über ein Kegelradgetriebe (17), das über ein Außengewinde (25) bei einer Drehung des Knopfes (16) den Ring (15) um die optische Achse (5) dreht.

Der Ring (15) enthält eine Steuernut (18), in die eine Rolle (19) eingreift. Bei einer Drehung des Knopfes (16) wird der Ring (15) gedreht. Über die Steuernut (18) und die Rolle (19) wird dabei die Fassung (7) in Richtung der optischen Achse (5) verschoben, wobei ein Fixierstift (20) in einer geraden Nut (21) eine Drehbewegung der Fassung verhindert.

Der Blenden-Einstellhebel (14) greift, wie aus Fig. 2 ersichtlich ist in eine Nut (22) im Ring (15) ein. Dadurch wird bei einer Betätigung des Drehknopfes (16) und der damit bewirkten Drehung des Ringes (15) der Betätigungshebel (14) geschwenkt und damit die Öffnung der Blende (13) verstellt.

Die in den Fig. 1 und 2 dargestellte Vorrichtung wird, wie Fig. 3 zeigt, zwischen dem Hauptobjektiv (12) und dem pankratischen System (23) des in Fig. 3 nur teilweise dargestellten Operationsmikroskopes befestigt.

Danach wird zunächst der Lichtleiter (2) durch Axialverschiebung seiner Fassung (3) so justiert, daß die gewünschte Ausleuchtung des Operationsfeldes erreicht wird. Je nach dem eingestellten Abstand der Lichtaustrittsfläche (11) von der Blende (13) wird das Operationsfeld gelichmäßig oder zum Beispiel mittenbetont ausgeleuchtet. Die einmal gewählte Art der Ausleuchtung des Operationsfeldes, wird durch Anziehen der Schraube (6) fixiert und bleibt bei der Axialverschiebung der Fassung (7) und damit des optischen Systems (8, 9) und der Blende (13) erhalten.

Zur Einstellung der Größe des ausgeleuchteten Feldes wird der Drehknopf (16) betätigt. Dadurch wird der Ring (15) gedreht und damit das optische System (8, 9) zusammen mit der Blende (13) in Richtung der optischen Achse (5) verschoben. Zugleich wird über den Einstellhebel (14) und die Nut (22) die Öffnung der Blende (13) verstellt.

Die Steuernut (18) kann so ausgebildet sein, daß zunächst nur die Öffnung der Blende (13) verstellt wird, während noch keine Axialverschiebung der Fassung (7) erfolgt. Ab einer vorbestimmten Blendenöffnung wird dann die Fassung (7) in Richtung der optischen Achse (5) verschoben und zugleich die Blendenöffnung verstellt.

Bei einer anderen Ausbildung der Steuernut (18) wird von vornherein die Blendenöffnung verstellt und zugleich die Fassung (7) axial verschoben. Durch entsprechende Ausbildung der Steuernut (18) lassen sich die Betätigung der Blende (13) und die Axialverschiebung der Fassung (7) in jeder gewünschten Weise kombinieren.

## Patentansprüche

1. Operationsmikroskop mit einer Beleuchtungseinrichtung, mit einem Hauptobjektiv (12) für die beiden stereoskopischen Beobachtungsstrahlengänge, mit einer Lichtquelle, welcher ein Lichtleiter (2) nachgeordnet ist, und mit einem optischen System, das aus einer Sammellinse (8) und einer asphärischen Linse (9) sowie einer Blende (13) besteht und zwischen dem Hauptobjektiv (12) und der Lichtaustrittsfläche (10) des Lichtleiters angeordnet ist, dadurch gekennzeichnet, daß das optische System entlang seiner optischen Achse verschiebbar ist, und daß eine Betätigungsvorrichtung (14-19) vorgesehen ist, welche die Axialverschiebung des Systems mit einer Öffnungsbewegung der Blende (13) kombiniert.

2. Operationsmikroskop nach Anspruch 1, dadurch gekennzeichnet, daß die Betätigungsvorrichtung (14-19) so ausgebildet ist, daß zunächst nur die Öffnung der Blende (13) verstellt wird, und ab einer vorbestimmten Blendenöffnung die Axialverschiebung der Fassung des optischen Systems erfolgt.

3. Operationsmikroskop nach Anspruch 1, dadurch gekennzeichnet, daß die Betätigungsvorrichtung so ausgebildet ist, daß die Blendenöffnung verstellt und zugleich die Fassung des optischen Systems axial verschoben wird.

4. Operationsmikroskop nach Anspruch 1 mit 3, dadurch gekennzeichnet, daß die Betätigungsvorrichtung (14 - 19) einen drehbaren Ring (15) enthält, der Steuernuten (18, 22) zur Axialverschiebung des Systems (8, 9, 13) und zur Betätigung der Blende (13) enthält.

5. Operationsmikroskop nach Anspruch 1, dadurch gekennzeichnet, daß zwischen dem Durchmesser der Öffnung der Blende (13) dem Verschiebeweg des Systems (8, 9, 13) und dem eingestellten Leuchtfelddurchmesser eine lineare Korrelation besteht.

6. Operationsmikroskop nach Anspruch 1, dadurch gekennzeichnet, daß zwischen dem Durchmesser der Öffnung der Blende (13) dem Verschiebeweg des Systems (8, 9, 13) und dem eingestellten Leuchtfelddurchmesser eine nichtlineare Korrelation besteht.

7. Operationsmikroskop nach Anspruch 1, dadurch gekennzeichnet, daß der Lichtleiter (2) axial verschiebbar zur Blende (13) angeordnet ist.

## Claims

1. Operation microscope with an illuminating device having a main objective (12) for the two stereoscopic observation ray paths and a source of light which is arranged behind a light guide (2) and an optical system which consists of a convergent lens (8) and an aspherical lens (9) and a diaphragm (13) which are arranged between the main objective (12) and the light guide exit surface, characterized by the fact that the optical system is displaceable along the optical axis and there is a actuating mechanism (14-19) which combines the displacement of the optical system with the opening movement of the diaphragm (13).

2. Operation microscope according to claim 1, characterized by the fact, that the actuating mechanism (14-19) is built in a way that at first only the diaphragm aperture varies and there results a displacement of the optical system along the optical axis from a certain diaphragm aperture.

3. Operation microscope according to claim 1, characterized by the fact, that the actuating mechanism (14-19) is built in a way that the diaphragm aperture changes while the optical system is displaced at the same time along the optical axis.

4. Operation microscope according to claim 1 and 3, characterized by the fact, that the actuating mechanism (14-19) comprises a rotatable ring (15) having cam means (18, 22) for displacement of the optical system (8, 9, 13) and to vary the diaphragm aperture.

5. Operation microscope according to claim 1, characterized by the fact, that a linear relationship exists between the diameter of the diaphragm aperture and the displacement of the optical system (8, 9, 13) and the adjusted diameter of the illuminating light.

6. Operation microscope according to claim 1, characterized by the fact, that a non-linear relationship exists between the diameter of the diaphragm aperture and the displacement of the optical system (8, 9, 13) and the adjusted diameter of the illuminating light.

7. Operation microscope according to claim 1, characterized by the fact, that the light guide (2) is axially displaceable with respect to the diaphragm (13).

## Revendications

1. Microscope d'opération avec un système d'éclairage, comprenant un objectif principal (12) couvrant les deux trajets lumineux d'observation stéréoscopique, une source de lumière dotée en aval d'un conducteur de lumière (2) et un système optique composé d'une lentille convergente (8), d'une lentille asphérique (9) et d'un diaphragme (13) et disposé entre l'objectif principal (12) et la sortie de lumière (10) du conducteur de lumière, caractérisé en ce que ledit système optique peut être déplacé le long de son axe optique et en ce qu'un dispositif d'actionnement (14-19) associe le déplacement axial du système à un mouvement d'ouverture du diaphragme (13).

2. Microscope d'opération selon la revendication 1, caractérisé en ce que le dispositif d'actionnement (14-19) est réalisé de façon à ce que seule l'ouverture du diaphagme (13) soit réglée dans un premier temps et que le déplacement axial de la monture du système optique ne se fasse qu'à partir d'un niveau d'ouverture prédéfini.

3. Microscope d'opération selon la revendication 1, caractérisé en ce que le dispositif d'actionnement est réalisé de façon à ce que le réglage du diaphagme (13) et le déplacement axial de la monture du système optique se fassent simultanément.

4. Microscope d'opération selon les revendications 1 et 3, caractérisé en ce que le dispositif d'actionnement (14-19) comprend une bague rotative (15) munie d'encoches (18, 22) commandant le déplacement axial du système (8, 9, 13) et le mouvement du diaphragme (13).

5. Microscope d'opération selon la revendication 1, caractérisé en ce que le diamètre d'ouverture du diaphragme (13), la translation du système (8, 9, 13) et le diamètre de champ lumineux réglé sont liés par une corrélation linéaire.

6. Microscope d'opération selon la revendication 1, caractérisé en ce que le diamètre d'ouverture du diaphragme (13), la translation du système (8, 9, 13) et le diamètre de champ lumineux réglé sont liés par une corrélation non linéaire.

7. Microscope d'opération selon la revendication 1, caractérisé en ce que le conducteur de lumière (2) est disposé de manière à pouvoir être déplacé axialement par rapport au diaphragme (13).
